# EUROPEAN PATENT APPLICATION

(11) **EP 1 396 248 A1**
(43) Date of publication of application: **10.03.2004**
(21) Application number: 02292193.6
(22) Date of filing: 06.09.2002
(51) Int. Cl.: A61H 37/00, A61F 7/00, A61H 1/00, A61H 15/02

(54) **Thermo-therapeutic mat**

(71) Applicant: Migun Medical Instrument Co., Ltd., Yeongi-Gun, Chungcheongnam-Do (KR)
(72) Inventor: Lee, Sang Bok, Teongi-Gun, Chungcheongnam-Do (KR)
(74) Representative: Lemoine, Robert

(57) **Abstract**

Disclosed is a thermo-therapeutic mat used for preventing and curing various diseases, and comprising removable upper and lower bodies, wherein the upper body comprises: sprockets rotatably fixed respectively to both sides of a central line traversing an upper cavity formed on the center of the upper body in a crosswise direction; a motor connected to either one of the sprockets; a chain for connecting the sprockets to each other; guide rails provided with uneven upper surfaces corresponding to vertebrae of a user, and installed on both edges of the upper cavity in parallel with the chain; shifters fixed to the chain, and horizontally reciprocating in the crosswise direction of the upper cavity according to the movement of the chain; fixing plates respectively installed on the corresponding shifters so as to vertically move up and down, and provided with traveling rollers respectively installed on both side surfaces of the fixing plate and moving along the guide rails; and therapeutic tools respectively fixed to the upper surfaces of the corresponding fixing plates, and horizontally reciprocating in the crosswise direction according to the movement of the chain and vertically moving up and down so as to massage and soothe affected parts of the upper part of the user's body. The therapeutic tools horizontally reciprocate in the crosswise direction and vertically move up and down, thereby effectively massaging and soothing the affected parts of the upper and lower parts of the user's body, being easily used, and improving its therapeutic effect.

## Description

### BACKGROUND OF THE INVENTION

### Field of the Invention

The present invention relates to a thermo-therapeutic mat, and more particularly to a thermo-therapeutic mat, which horizontally reciprocates therapeutic tools in the crosswise direction and vertically moves therapeutic tools up and down, thereby effectively massaging and soothing affected parts of the body of a user, and thus being conveniently used and improving its therapeutic effect.

### Description of the Related Art

As well known to those skilled in the art, various physical-therapeutic devices such as a thermo-therapeutic device and a high frequency therapeutic device are used at home. The thermo-therapeutic device massages and soothes affected parts of a user using a helium ramp, an infrared ramp, or etc. On the other hand, the high frequency therapeutic device uses a high-frequency ramp.

In order to use the aforementioned conventional thermo-therapeutic device or high frequency therapeutic device, the thermo-therapeutic device or the high frequency therapeutic device must be exactly located on a spot of a vertebra of the affected part of the user, and then irradiate light or high frequency vibration on the spot of the vertebra of the user for a designated time. Next, the thermo-therapeutic device or the high frequency therapeutic device is transferred into another vertebra of the user, and then also irradiates light or high frequency vibration on this vertebra.

However, the user sets the above-described conventional thermo-therapeutic device or high frequency therapeutic device directly on a spot of his/her vertebra desired to be treated. After a designated time, the user transfers the thermo-therapeutic device or the high frequency therapeutic device to a spot of another vertebra desired to be treated. Therefore, the conventional physical therapeutic devices cause the user inconvenience. Further, in case that the user does not set the physical therapeutic device to an exact position of a spot of the vertebra desired to be treated, the physical therapeutic device is ineffective.

### SUMMARY OF THE INVENTION

Therefore, the present invention has been made in view of the above problems, and it is an object of the present invention to provide a thermo-therapeutic mat, which horizontally reciprocates therapeutic tools in the crosswise direction and vertically moves therapeutic tools up and down, thereby effectively massaging and soothing affected parts of the body of a user, and thus being conveniently used and improving its therapeutic effect.

In accordance with the present invention, the above and other objects can be accomplished by the provision of a thermo-therapeutic mat used for preventing and curing various diseases, and comprising removable upper and lower bodies, wherein the upper body comprises: sprockets rotatably fixed respectively to both sides of a central line traversing an upper cavity formed on the center of the upper body in a crosswise direction; a motor connected to either one of the sprockets; a chain for connecting the sprockets to each other; guide rails provided with uneven upper surfaces corresponding to vertebrae of a user, and installed on both edges of the upper cavity in parallel with the chain; shifters fixed to the chain, and horizontally reciprocating in the crosswise direction of the upper cavity according to the movement of the chain; fixing plates respectively installed on the corresponding shifters so as to vertically move up and down, and provided with traveling rollers respectively installed on both side surfaces of the fixing plate and moving along the guide rails; and therapeutic tools respectively fixed to the upper surfaces of the corresponding fixing plates, and horizontally reciprocating in the crosswise direction according to the movement of the chain and vertically moving up and down so as to massage and soothe affected parts of the upper part of the user's body.

Preferably, a plurality of guide rods for connecting the fixing plate to the shifter and guiding the vertical movement of the fixing plate may be formed on the lower surface of the fixing plate, and a plurality of connection holes for receiving the corresponding guide rods may be formed on the upper surface of the shifter so that the guide rods vertically move up and down.

Further, preferably, a guide groove may be formed on each outer side surface of the guide rails, and a guide roller inserted into the guide groove and moving along the guide groove may be installed on both side surfaces of the shifter.

The lower body of the thermo-therapeutic mat of the present invention may comprise: sprockets rotatably fixed respectively to both sides of a central line traversing a lower cavity formed on the center of the lower body in a crosswise direction; a movable lever connected to either one of the sprockets via a rotary axis; a chain for connecting the sprockets to each other; a guide rail formed in the crosswise direction between the sprockets; a mounting plate connected to the chain and installed on the upper surface of the guide rail so as to slide along the guide rail; therapeutic tools respectively fixed to the upper surface of the mounting plate and horizontally reciprocating in the crosswise direction according to the movement of the chain so as to massage and soothe affected parts of the lower part of the user's body; and a space for accommodating therapeutic accessories including the therapeutic tools and a remote control.

### BRIEF DESCRIPTION OF THE DRAWINGS

The above and other objects, features and other advantages of the present invention will be more clearly understood from the following detailed description taken in conjunction with the accompanying drawings, in which:
Fig. 1 is a perspective view of a thermo-therapeutic mat in accordance with an embodiment of the present invention;
Fig. 2 is a perspective view of the thermo-therapeutic mat of Fig. 1 without an outer cover;
Fig. 3 is an exploded perspective view of an upper body of the thermo-therapeutic mat of Fig. 1;
Fig. 4 is a top view of the upper body of the thermo-therapeutic mat of Fig. 1;
Fig. 5 is a cross-sectional view of the upper body of the thermo-therapeutic mat of Fig. 1;
Figs. 6a and 6b are longitudinal-sectional views of the upper body of the thermo-therapeutic mat of Fig. 1;
Fig. 7 is an exploded perspective view of a lower body of the thermo-therapeutic mat of Fig. 1;
Fig. 8 is a cross-sectional view of the lower body of the thermo-therapeutic mat of Fig. 1;
Fig. 9 is a longitudinal-sectional view of the lower body of the thermo-therapeutic mat of Fig. 1;
Fig. 10 is a top view of an upper body of a thermo-therapeutic mat in accordance with another embodiment of the present invention without an outer cover;
Fig. 11 is a cross-sectional view of the upper body of the thermo-therapeutic mat of Fig. 10;
Fig. 12 is a top view of an upper body of a thermo-therapeutic mat in accordance with yet another embodiment of the present invention without an outer cover; and
Fig. 13 is a cross-sectional view of the upper body of the thermo-therapeutic mat of Fig. 12.

### DESCRIPTION OF THE PREFERRED EMBODIMENTS

Now, preferred embodiments of the present invention will be described in detail with reference to the annexed drawings.

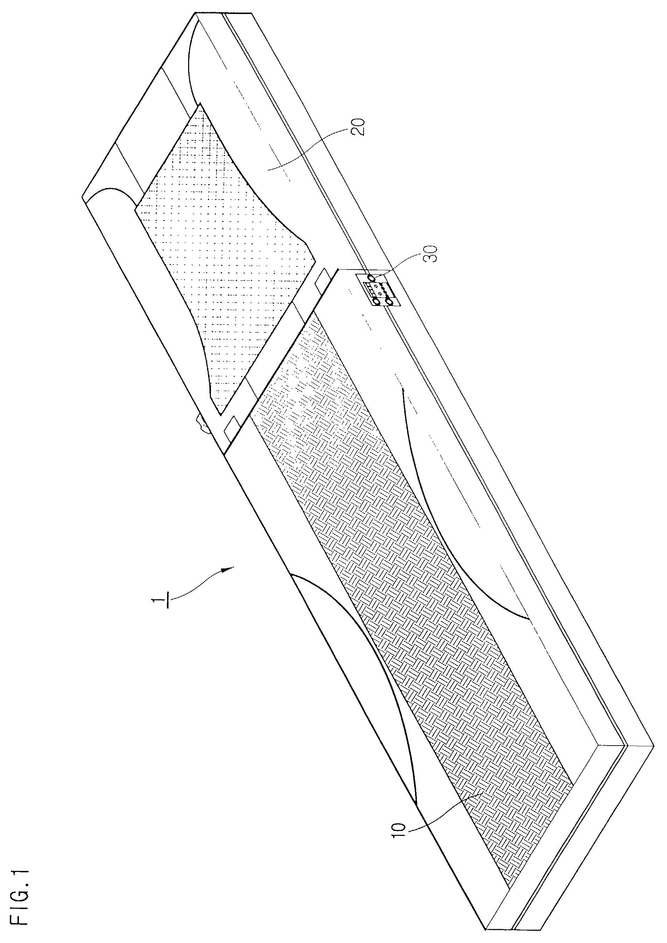

Fig. 1 is a perspective view of a thermo-therapeutic mat in accordance with an embodiment of the present invention. The thermo-therapeutic mat 1 of the first embodiment of the present invention comprises an upper body 10 for receiving the upper part of the human body and a lower body 20 for receiving the lower part of the human body. The upper and lower bodies 10 and 20 of the thermo-therapeutic mat 1 are removable. Further, the upper and lower bodies 10 and 20 are respectively coated with a removable outer cover made of leather, cloth, or etc. A controlling unit 30 is installed on a side surface of the upper body 10. The controlling unit 30 serves to operate therapeutic tools for massaging and soothing affected parts of the user and set the temperature of the therapeutic tools.

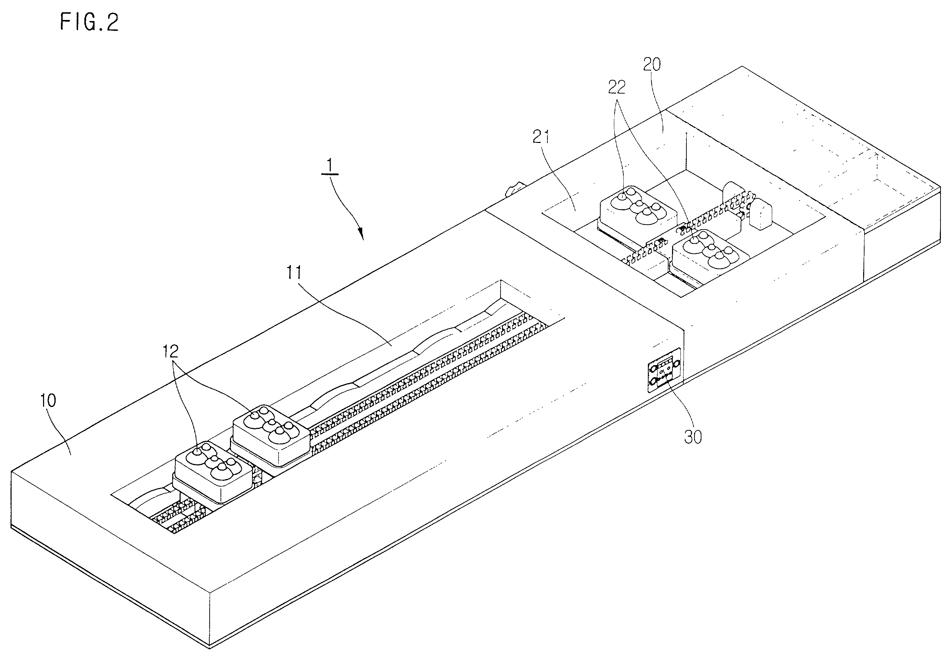

Fig. 2 is a perspective view of the thermo-therapeutic mat 1 of Fig. 1 without an outer cover. An upper cavity 11 for accommodating therapeutic tools 12 for massaging and soothing affected parts of the upper part of the user's body is formed on the center of the upper body 10 of the thermo-therapeutic mat 1. A lower cavity 21 for accommodating therapeutic tools 22 for massaging and soothing affected parts of the lower part of the user's body is formed on the center of the lower body 20 of the thermo-therapeutic mat 1. The therapeutic tools 12 of the upper body 10 and the therapeutic tools 22 of the lower body 20 horizontally reciprocate in the crosswise direction and vertically move up and down.

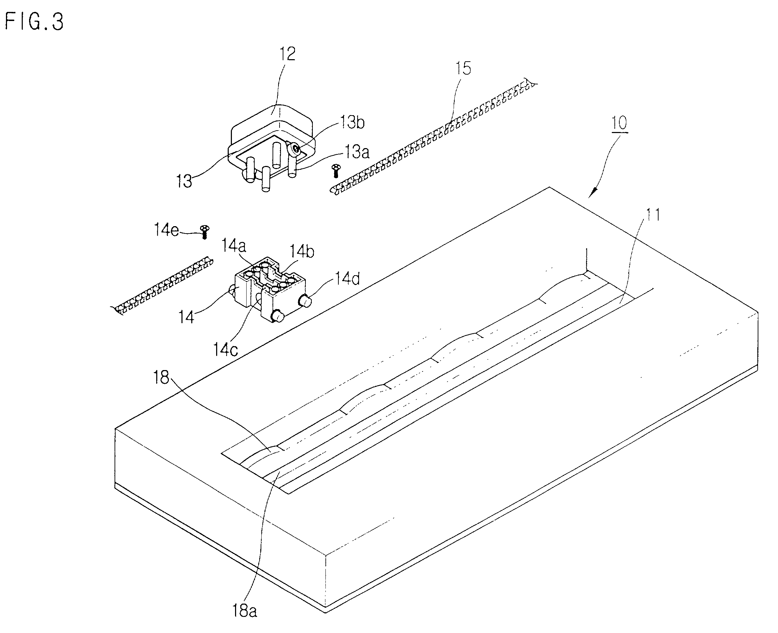

Hereinafter, with reference to Figs. 3 to 9, the reciprocation motion and the vertical movement of the therapeutic tools 12 and 22 will de described in detail.

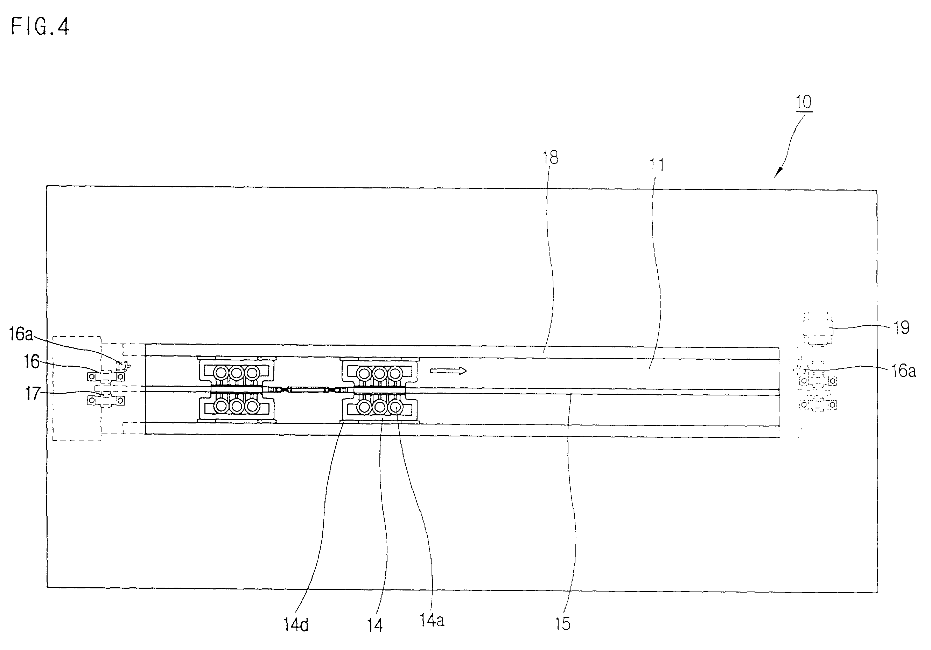

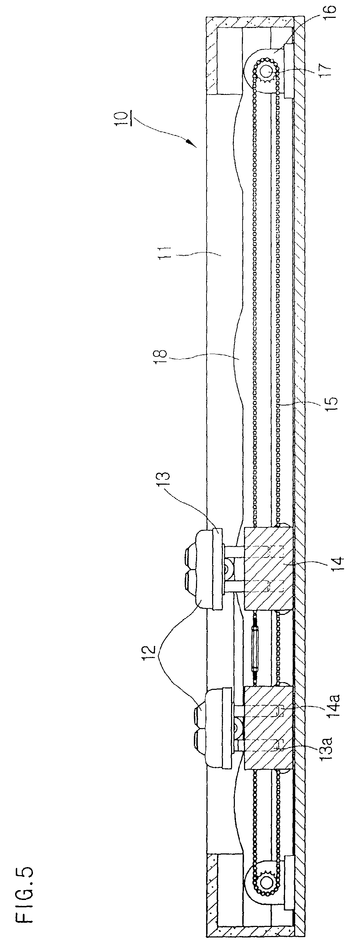

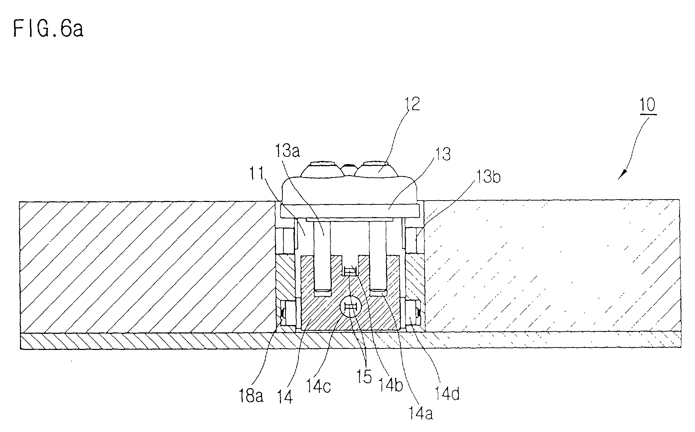

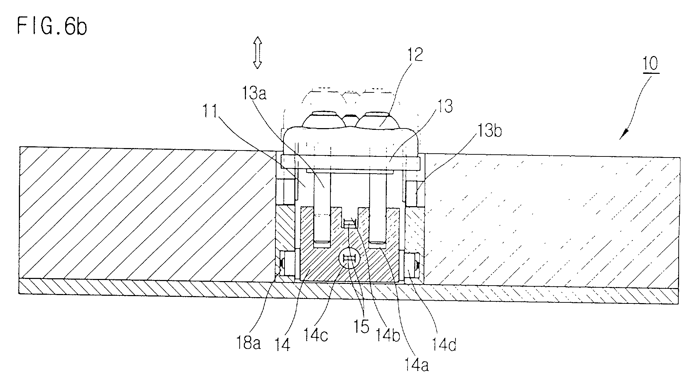

Figs. 3 to 6 shows an installed state of the therapeutic tools 12 for massaging and soothing affected parts of the upper part of the user's body on the upper body 10 of the thermo-therapeutic mat 1 in accordance with the first embodiment of the present invention. More particularly, Fig. 3 is an exploded perspective view of the upper body 10 of the thermo-therapeutic mat 1. Fig. 4 is a top view of the upper body 10 of the thermo-therapeutic mat 1 without the therapeutic tools 12. Fig. 5 is a cross-sectional view of the upper body 10 of the thermo-therapeutic mat 1. Figs. 6a and 6b are longitudinal-sectional views of the upper body 10 of the thermo-therapeutic mat 1.

As shown in Figs. 3 to 6, a sprocket 17 supported by a fixed bracket 16 is formed on both sides of a central line traversing the upper cavity 11 formed on the center of the upper body 10 in the crosswise direction. A rotary axis of a motor 19 is connected to the sprocket 17 formed on one side. Therefore, a chain 15 for connecting one sprocket 17 to the other sprocket 17 moves in the crosswise direction of the thermo-therapeutic mat 1 by the reciprocal rotation of the motor 19, i.e., the regular and reverse rotation of the motor 19.

A guide rail 18 is respectively formed on both edges of the upper cavity 11 in parallel with the chain 15. The upper surfaces of the both guide rails 18 are uneven, thereby corresponding to the vertebrae of the user. A guide groove 18a is formed on a designated position of the outer side surface of the both guide rails 18 in the crosswise direction.

The therapeutic tool 12 for massaging and soothing the affected parts of the user's body is fixed to an upper surface of a fixing plate 13. The fixing plate 13 is connected to a shifter 14. The shifter 14 reciprocates in the crosswise direction within the upper cavity 11 by the movement of the chain 15.

That is, a plurality of guide rods 13a are formed on the lower surface of the fixing plate 13. The guide rods 13a serve to connect the fixing plate 13 to the shifter 14, and to guide the vertical movement of the fixing plate 13. A traveling roller 13b is installed on both sides of the fixing plate 13. The traveling rollers 13b travel along the corresponding guide rails 18.

A plurality of connection holes 14a are formed on the upper surface of the shifter 14. The guide rod 13a of the fixing plate 13 is inserted into the corresponding connection hole 14a of the shifter 14 so that the guide rod 13a vertically moves up and down. A fixing groove 14b for fixing the chain 15 is formed on the center of the upper surface of the shifter 14. A through hole 14c is formed on the shifter 14 in the crosswire direction, and the chain 15 penetrates the shifter 14 via the through hole 14c. Further, a guide roller 14d is installed on both side surfaces of the shifter 14, thereby being inserted into the corresponding guide groove 18a on the side surface of the guide rail 18 and moving along the guide groove 18a.

The guide rollers 14d of the shifter 14 are inserted into the corresponding guide grooves 18a of the guide rails 18. The chain 15 is inserted into the through hole 14c of the shifter 14, and connected to the respective sprockets 17. Then, the both terminals of the chain 15 are respectively fixed to the fixing grooves 14b of the shifters 14 by fixtures 14e. Thereby, the shifters 14 move along the guide rails 18 in the crosswise direction by the movement of the chain 15.

When the guide rods 13a of the fixing plate 13 provided with the therapeutic tool 12 for massaging and soothing the affected parts of the user's body are inserted into the connection holes 14a of the shifter 14, the traveling rollers 13b of the fixing plate 13 are disposed on the upper surfaces of the guide rails 18. Therefore, as described above, when the shifters 14 move along the guide rails 18 in the crosswise direction by the movement of the chain 15, the traveling rollers 13b of the fixing plate 13 travel along the uneven upper surfaces of the guide rails 18. Then, as shown in Figs. 6a and 6b, the fixing plate 13 provided with the therapeutic tool 12 vertically moves up and down, thereby massaging and soothing vertebrae of the affected parts of the user's body.

Herein, a reference numeral 16a denotes limiting means for limiting a moving distance of the shifter 14.

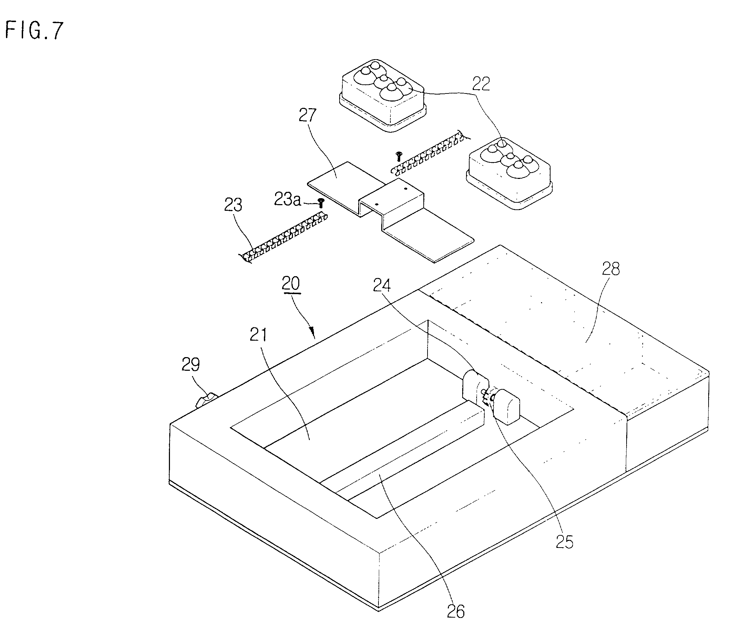

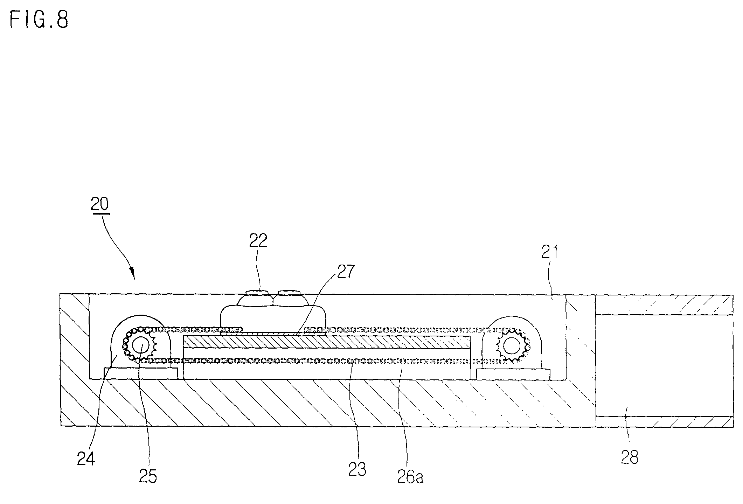

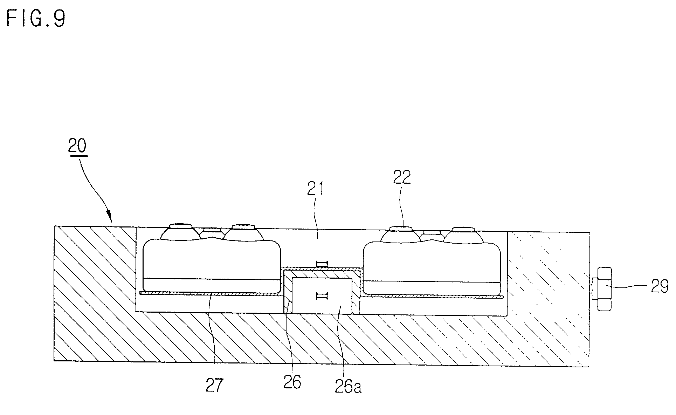

Figs. 7 to 9 show an installed state of the lower body 22 of the thermo-therapeutic mat 1. More particularly, Fig. 7 is an exploded perspective view of the lower body 22, and Fig. 8 is a cross-sectional view of the lower body 22. Fig. 9 is a longitudinal-sectional view of the lower body 22.

As shown in Figs. 7 to 9, a sprocket 25 supported by a fixed bracket 24 is formed on both sides of a central line traversing the lower cavity 21 formed on the center of the lower body 20 in a crosswise direction. A movable lever 29 is connected to the sprocket 25 formed on one side via a rotary axis (not shown).

A guide rail 26 is formed on the lower cavity 21 in the crosswise direction between the both sprockets 25. A through hole 26a is formed on the lower surface of the guide rail 26 in the crosswise direction. A mounting plate 27 for fixing the therapeutic tools 22 for massaging and soothing affected parts of the lower part of the user's body is installed on the upper surface of the guide rail 26 so that the mounting plate 27 slides along the guide rail 26.

That is, a chain 23 is inserted into the through hole 26a formed on the lower surface of the guide rail 26 and connected to the sprockets 25. The mounting plate 27 provided with the therapeutic tools 22 is installed on the guide rail 26 disposed between the both sprockets 25. The both terminals of the chain 23 are respectively fixed to the mounting plate 27 by fixtures 23a. Then, the mounting plate 27 moves along the guide rail 26 in the crosswise direction by the movement of the chain 23, thereby massaging and soothing the affected parts of the lower part of the user's body.

The lower body 20 further comprises a space 28 for accommodating therapeutic accessories including the therapeutic tools 12 and 22, a remote control, etc., thereby preventing the loss of the therapeutic accessories and safely storing and managing the therapeutic accessories.

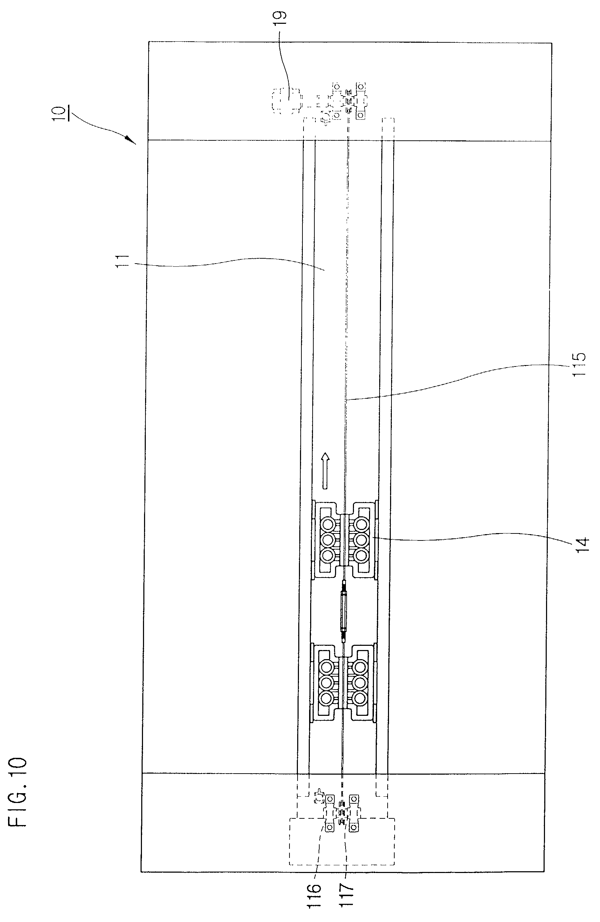

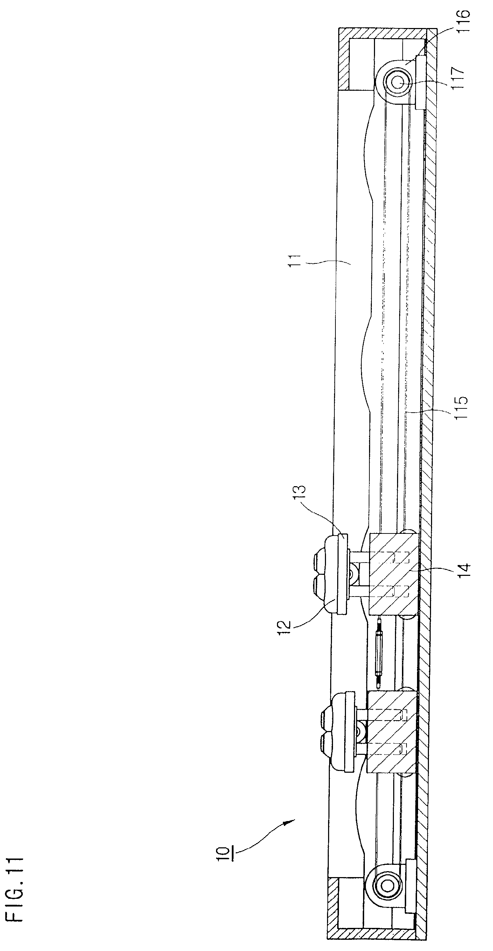

Figs. 10 and 11 show a thermo-therapeutic mat in accordance with a second embodiment of the present invention. More particularly, Fig. 10 is a top view of an upper body of the thermo-therapeutic mat without an outer cover and therapeutic tools, and Fig. 11 is a cross-sectional view of the upper body of the thermo-therapeutic mat.

As shown in Figs. 10 and 11, the thermo-therapeutic mat of the second embodiment of the present invention has the same constitution as that of the first embodiment of the present invention except that the shifters 14 horizontally move in the crosswise direction using a wire 115. That is, a pulley 117 is fixed to both sides of a central line traversing the upper cavity 11 formed on the center of the upper body 10 in a crosswise direction, and the wire 115 is connected to the pulleys 117. Therefore, the shifters 14 and the fixing plates 13 provided with the therapeutic tools 12 move in the crosswise direction of the thermo-therapeutic mat 1 by the driving of the motor 19.

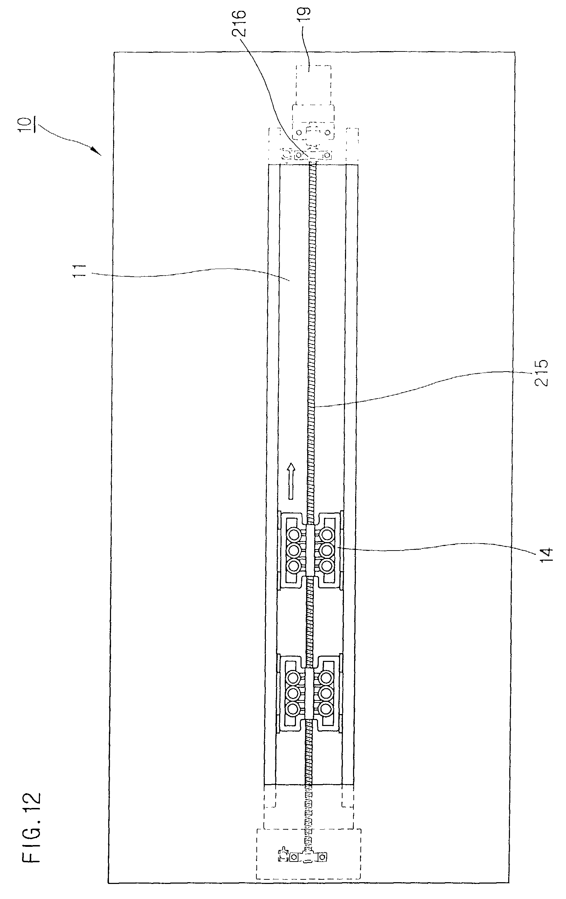

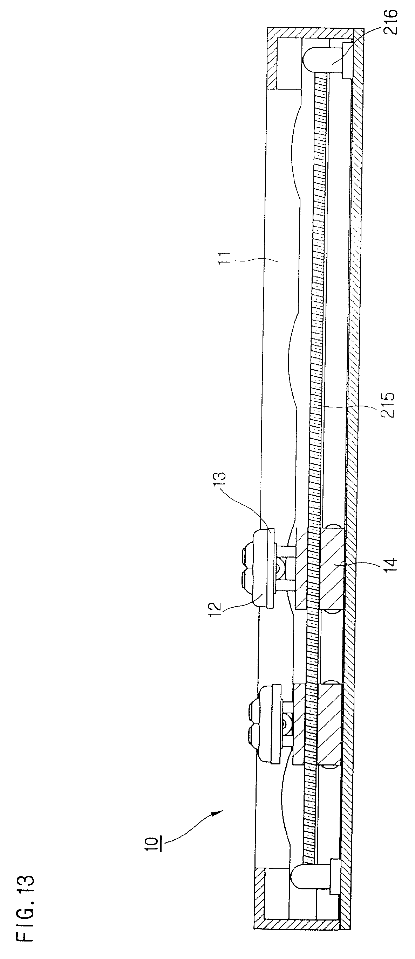

Figs. 12 and 13 show a thermo-therapeutic mat in accordance with a third embodiment of the present invention. More particularly, Fig. 12 is a top view of an upper body of the thermo-therapeutic mat without an outer cover and therapeutic tools, and Fig. 13 is a cross-sectional view of the upper body of the thermo-therapeutic mat.

As shown in Figs. 12 and 13, the thermo-therapeutic mat of the third embodiment of the present invention has the same constitution as that of the first embodiment of the present invention except that the shifters 14 are spirally connected to a feed shaft 215 and move in the crosswise direction. That is, the feed shaft 215 is rotatably connected to fixed brackets 216 respectively formed on both sides of a central line traversing the upper cavity 11 formed on the center of the upper body 10 in the crosswise direction, and one terminal of the feed shaft 215 is connected to a rotary axis of the motor 19. Therefore, the feed shaft 215 rotates by the driving of the motor 19, and thus the shifters 14 and the fixing plates 13 provided with the therapeutic tools 12 move in the crosswise direction of the thermo-therapeutic mat 1.

In the aforementioned second and third embodiments of the present invention, only the operation of the therapeutic tools 12 in the upper body 10 of the thermo-therapeutic mat 1 is described. However, in the second and third embodiments of the present invention, the therapeutic tools 22 in the lower body 20 of the thermo-therapeutic mat 1 also move in the crosswise direction of the thermo-therapeutic mat 1 using the wire 115 and the feed shaft 215, respectively.

As apparent from the above description, the present invention provides a thermo-therapeutic mat, which horizontally reciprocates therapeutic tools in the crosswise direction and vertically moves therapeutic tools up and down, thereby effectively massaging and soothing affected parts of the upper and lower parts of the user's body, and thus being conveniently used and improving its therapeutic effect. Further, the thermo-therapeutic mat of the present invention comprises a space for accommodating therapeutic accessories including the therapeutic tools, a remote control, etc., thereby safely storing and managing the therapeutic accessories.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A thermo-therapeutic mat used for preventing and curing various diseases, and comprising removable upper and lower bodies, wherein the upper body comprises:
sprockets rotatably fixed respectively to both sides of a central line traversing an upper cavity formed on the center of the upper body in a crosswise direction;
a motor connected to either one of the sprockets;
a chain for connecting the sprockets to each other;
guide rails provided with uneven upper surfaces corresponding to vertebrae of a user, and installed on both edges of the upper cavity in parallel with the chain;
shifters fixed to the chain, and horizontally reciprocating in the crosswise direction of the upper cavity according to the movement of the chain;
fixing plates respectively installed on the corresponding shifters so as to vertically move up and down, and provided with traveling rollers respectively installed on both side surfaces of the fixing plate and moving along the guide rails; and
therapeutic tools respectively fixed to the upper surfaces of the corresponding fixing plates, and horizontally reciprocating in the crosswise direction according to the movement of the chain and vertically moving up and down so as to massage and soothe affected parts of the upper part of the user's body.

2. The thermo-therapeutic mat as set forth in claim 1, wherein a plurality of guide rods for connecting the fixing plate to the shifter and guiding the vertical movement of the fixing plate are formed on the lower surface of the fixing plate, and a plurality of connection holes for receiving the corresponding guide rods are formed on the upper surface of the shifter so that the guide rods vertically move up and down.

3. The thermo-therapeutic mat as set forth in claim 1, wherein a guide groove is formed on each outer side surface of the guide rails, and a guide roller inserted into the guide groove and moving along the guide groove is installed on both side surfaces of the shifter.

4. A thermo-therapeutic mat used for preventing and curing various diseases, and comprising removable upper and lower bodies, wherein the upper body comprises:
pulleys rotatably fixed respectively to both sides of a central line traversing an upper cavity formed on the center of the upper body in a crosswise direction;
a motor connected to either one of the pulleys;
a wire for connecting the pulleys to each other;
guide rails provided with uneven upper surfaces corresponding to vertebrae of a user, and installed on both edges of the upper cavity in parallel with the wire;
shifters fixed to the wire, and horizontally reciprocating in the crosswise direction of the upper cavity according to the movement of the wire;
fixing plates respectively installed on the corresponding shifters so as to vertically move up and down, and provided with traveling rollers respectively installed on both side surfaces of the fixing plate and moving along the guide rails; and
therapeutic tools respectively fixed to the upper surfaces of the corresponding fixing plates, and horizontally reciprocating in the crosswise direction according to the movement of the wire and vertically moving up and down so as to massage and soothe affected parts of the upper part of the user's body.

5. A thermo-therapeutic mat used for preventing and curing various diseases, and comprising removable upper and lower bodies, wherein the upper body comprises:
a feed shaft with both terminals rotatably fixed to fixed brackets respectively formed on both sides of a central line traversing an upper cavity formed on the center of the upper body in a crosswise direction;
a motor connected to one terminal of the feed shaft;
guide rails provided with uneven upper surfaces corresponding to vertebrae of a user, and installed on both edges of the upper cavity in parallel with the feed shaft;
shifters spirally connected to the feed shaft, and horizontally reciprocating in the crosswise direction of the upper cavity according to the rotation of the feed shaft;
fixing plates respectively installed on the corresponding shifters so as to vertically move up and down, and provided with traveling rollers respectively installed on both side surfaces of the fixing plate and moving along the guide rails; and
therapeutic tools respectively fixed to the upper surfaces of the corresponding fixing plates, and horizontally reciprocating in the crosswise direction according to the movement of the wire and vertically moving up and down so as to massage and soothe affected parts of the upper part of the user's body.

6. The thermo-therapeutic mat as set forth in claim 1, 4, or 5, wherein the lower body comprises:
sprockets rotatably fixed respectively to both sides of a central line traversing a lower cavity formed on the center of the lower body in a crosswise direction;
a movable lever connected to either one of the sprockets via a rotary axis;
a chain for connecting the sprockets to each other;
a guide rail formed in the crosswise direction between the sprockets;
a mounting plate connected to the chain and installed on the upper surface of the guide rail so as to slide along the guide rail;
therapeutic tools respectively fixed to the upper surface of the mounting plate and horizontally reciprocating in the crosswise direction according to the movement of the chain so as to massage and soothe affected parts of the lower part of the user's body; and
a space for accommodating therapeutic accessories including the therapeutic tools and a remote control.
